(19) Europäisches Patentamt
European Patent Office
Office européen des brevets

(11) **EP 4 312 743 B1**

(12) **EUROPEAN PATENT SPECIFICATION**

(45) Date of publication and mention
of the grant of the patent:
**06.05.2026 Bulletin 2026/19**

(21) Application number: **22719408.1**

(22) Date of filing: **31.03.2022**

(51) International Patent Classification (IPC):
***A61B 5/026*** (2006.01)    ***G06T 3/40*** (2024.01)

(52) Cooperative Patent Classification (CPC):
**A61B 5/0261; G06T 7/0016;** G06T 2207/10016;
G06T 2207/30104

(86) International application number:
**PCT/US2022/022734**

(87) International publication number:
**WO 2022/212636 (06.10.2022 Gazette 2022/40)**

(54) **SYNTHETIC MULTI-EXPOSURE SPECKLE IMAGING (SYMESI) METHOD**

VERFAHREN FÜR SYNTHETISCHE MEHRFACHBELICHTUNGS-SPECKLE-BILDGEBUNG (SYMESI)

PROCÉDÉ D'IMAGERIE DE GRANULARITÉ MULTI-EXPOSITION SYNTHÉTIQUE (SYMESI)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.04.2021 US 202163200914 P**

(43) Date of publication of application:
**07.02.2024 Bulletin 2024/06**

(73) Proprietor: **University of South Florida
Tampa, FL 33612-9220 (US)**

(72) Inventors:
• **PARTHASARATHY, Ashwin Bharadwaj
Tampa, Florida 33647 (US)**
• **SAFI, Abdul Mohaimen
Tampa, Florida 33612 (US)**

(74) Representative: **FRKelly
Waterways House
Grand Canal Quay
Dublin D02 PD39 (IE)**

(56) References cited:
**US-A1- 2018 012 337**

• **SAFI ABDUL MOHAIMEN ET AL: "Quantitative
Cerebral Blood Flow Imaging with Synthetic
Single-Shot Multi-Exposure Laser Speckle
Imaging", BIOPHOTONICS CONGRESS 2021,
WASHINGTON, DC UNITED STATES 12-16 APRIL
2021, 1 January 2021 (2021-01-01), Washington,
D.C., pages 1 - 3, XP093210986, ISBN:
978-1-943580-85-9, DOI: 10.1364/
BRAIN.2021.BW3B.4**
• **SAFI ABDUL MOHAIMEN: "Quantitative Cerebral
Blood Flow Imaging with Synthetic Single-Shot
Multi-Exposure Laser Speckle Imaging, video
presentation, slide 14", 16 April 2021
(2021-04-16), pages 1 - 1, XP093279467,
Retrieved from the Internet <URL:https://opg.
optica.org/abstract.cfm?
uri=BRAIN-2021-BW3B.4>**
• **DRAGOJEVIC TANJA ET AL: "Compact, multi-
exposure speckle contrast optical spectroscopy
(SCOS) device for measuring deep tissue blood
flow", BIOMEDICAL OPTICS EXPRESS, vol. 9,
no. 1, 1 January 2018 (2018-01-01), United States,
pages 322, XP055937568, ISSN: 2156-7085, DOI:
10.1364/BOE.9.000322**

- **SUN SHEN ET AL: "Multi-exposure laser speckle contrast imaging using a high frame rate CMOS sensor with a field programmable gate array", OPTICS LETTERS, vol. 40, no. 20, 15 October 2015 (2015-10-15), US, pages 4587, XP055937569, ISSN: 0146-9592, DOI: 10.1364/OL.40.004587**
- **HULTMAN MARTIN ET AL: "Real-time video-rate perfusion imaging using multi-exposure laser speckle contrast imaging and machine learning", JOURNAL OF BIOMEDICAL OPTICS, SPIE, 1000 20TH ST. BELLINGHAM WA 98225-6705 USA, vol. 25, no. 11, 1 November 2020 (2020-11-01), pages 116007, XP060154011, ISSN: 1083-3668, [retrieved on 20201115], DOI: 10.1117/1.JBO.25.11.116007**

## Description

CROSS-REFERENCE TO RELATED APPLICATIONS

This international patent application claims priority from and benefit of the US Provisional Patent Application No. 63/200,914 filed on April 02, 2021.

RELATED ART

Blood flow through tissue serves as an important physiological index of health of such tissue, because blood flow directly indicates oxygen delivery to the tissue and is critical for normal tissue functioning. Since even small changes in oxygen supply to the brain, for example, can have a dramatic impact on normal physiological processes, reduction in blood flow to the brain is not without serious consequences. Therefore, imaging of blood flow is important to understand the normal functioning of physiology, monitor disease progression, and to track treatment.

Generally, superior spatiotemporal resolution characteristics of optical imaging methods make them more suitable for visualizing cerebral blood flow (CBF) dynamics than, for example, Magnetic Resonance Imaging (MRI), Computed Tomography (CT), or Diffuse Optical Tomography (DOT) - especially for applications that require resolution of individual cerebral blood vessels. Optical imaging methodologies rooted in photon correlation, for example (such as Laser Speckle Contrast Imaging, or LSCI) are particularly well suited for assessing the CBF with the use of intrinsically contrast motion of red blood cells.

LSCI, in particular, has been shown useful for imaging cerebral blood flow in small animal models. One of primary advantages of LSCI is its ability to obtain wide-field CBF images with superior spatial and temporal resolution while the employed imaging apparatus is simple and inexpensive. To date, LSCI has been utilized to image CBF dynamics during ischemia in rat and mouse brains, in functional activation studies, and to model ischemic stroke progression.

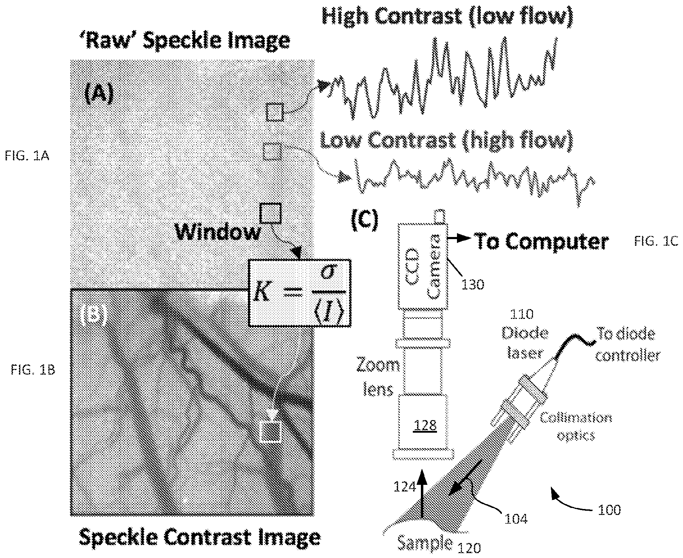

As is recognized in related art, laser speckle is the random interference pattern produced by coherent addition of light fields (for example, of laser light fields) that have backscattered from a sample along trajectories with slightly different path lengths. To this end, FIG. 1C schematically shows a typical LSCI setup. Here, slightly divergent light 104 from a visible or near infrared diode laser light source 110 is directed to impinge on the tissue sample 120; light 104 scatters in the tissue while the optical field components travelling along different paths through the tissue 120 experience different phase shifts. The backscattered light 124 is collected through an imaging lens 128 and recorded, in the form of a speckle, on the camera sensor 130 for data processing at the appropriately configured electronic circuitry 130.

The movements of particles (such as, for example, red blood cells) in the sample, cells, impress spatial and temporal fluctuations onto the speckle pattern (see, for example, Ref. 3). Such effect manifests as local blurring (or decorrelation) in the image (FIG. 1A). Quantification of this blurring or decorrelation provides a measure of flow of red blood cells in the vasculature of the tissue 120. Formally, the local speckle contrast, $K = \sigma_s/\langle I \rangle$, *i.e.,* the square root of normalized variance of local intensity, is computed in a small window (usually 7×7 pixels) within the image; here, $\sigma_s$ is the standard deviation of the intensities in the 7x7 pixel window, and (I) is the mean of intensities in the 7x7 pixel window. The processed speckle contrast image (FIG. 1B) is then produced by repeating this computation, window-by-window, across the raw speckle image. The higher value of speckle contrast in a given portion of the image of FIG. 1B is a manifestation of the reduced amount of spatial blurring in the corresponding region of the sample *(i.e.,* in FIG. 1A), which is a direct consequence of slow-moving red blood cells.

Notably, as recognized in related art, speckle contrast measured with the LSCI approach is appropriate only for acute measurements of *relative* changes of the CBF changes. In other words, the results of practical use of the conventional implementation of the LSCI system does not allow for quantitative measurement of movement at or in the target tissue, producing instead assessment of the relative (for example, on the scale of arbitrary units from 0 to 1) contrast values across the speckle image that manifests only in and affords only one resulting conclusion about the CBF changes, which can be expressed as follows: a movement of the target particles (say, blood flow) at the first region of the target tissue depicted in the first portion of the image is occurring quicker than a movement of the target particles at the second region of the target tissue depicted in the second portion of the image. The quantitative (that is, numerical) assessment of the speed values of these two movements are simply not possible based on the conventional LSCI, which provides relative - that is, comparative - results. As a result, the application of the conventional LSCI system has been limited to only the very simplest of situations.

The methodology known as Multi-Exposure Speckle Imaging (or, MESI) was introduced to correct the inability of the conventional LSCI to provide quantitative measurements of blood flow by processing laser speckle images acquired at multiple camera exposures and fitting them to a quantitative speckle visibility model. To-date, MESI has been accomplished by either time-gating the laser using relatively bulky and slow instrumentation such as an acousto-optic modulator or a rotating filter wheel (see, e.g., Parthasarathy et al., " Robust flow measurement with multi-exposure speckle imaging," Optics Express 16, 1975-1989; 2008) or using temporal binning approach (albeit with an expensive fast detector better suited for low-resolution images, such as 8x5 pixels, only; see, e.g., Ref. G). The well-recognized shortcomings of the MESI approach manifests in the need to use of dedicated piece of hardware taking care of maintaining the useful laser output power constant at the sample (tissue) being interrogated and/or duration of the measurements that is not short enough for multiple practical applications.

Cited References:

- Ref. A Parthasarathy, A. B., Kazmi, S. M. S. & Dunn, A. K. Quantitative imaging of ischemic stroke through thinned skull in mice with Multi Exposure Speckle Imaging. Biomedical optics express 1, 246-259, doi:10.1364/BOE.1.000246 (2010).
- Ref. B Kazmi, S. M. S., Parthasarathy, A. B., Song, N. E., Jones, T. A. & Dunn, A. K. Chronic imaging of cortical blood flow using Multi-Exposure Speckle Imaging. Journal of Cerebral Blood Flow & Metabolism 33, 798-808, doi: 10.1038/jcbfm.2013.57 (2013).
- Ref. C Parthasarathy, A. B., Tom, W. J., Gopal, A., Zhang, X. & Dunn, A. K. Robust flow measurement with multi-exposure speckle imaging. Optics Express 16, 1975-1989 (2008).
- Ref. D Richards, L. M. et al. Intraoperative multi-exposure speckle imaging of cerebral blood flow. Journal of Cerebral Blood Flow & Metabolism 37, 3097-3109, doi:10.1177/10271678X16686987 (2017).
- Ref. E Valdes, C. P. et al. Speckle contrast optical spectroscopy, a non-invasive, diffuse optical method for measuring microvascular blood flow in tissue. Biomedical optics express 5, 2769, doi:10.1364/BOE.5.002769 (2014).
- Ref. F Varma, H. M., Valdes, C. P., Adams, K. E., Culver, J. P. & Durduran, T. Speckle contrast optical tomography: A new method for deep tissue three-dimensional tomography of blood flow. Biomedical optics express 5, 1275, doi:10.1364/BOE.5.001275 (2014).
- Ref. G Dragojević et al., "High-speed multi-exposure laser speckle contrast imaging with a single-photon counting camera," Biomedical Optics Express 6, 2865-2876; 2015).

Another prior art speckle method is known from Dragojevic et al., "Compact, multi-exposure speckle contrast optical spectroscopy (SCOS) device for measuring deep tissue blood flow. "Biomedical optics express 9, 322, doi: 10.1364/BOE.9.000322 (2018).

SUMMARY

The present disclosure provides a multiple-synthetic-exposure-time speckle imaging (syMESI) system that includes an optical illumination system, an optical imaging (or light collecting) system, and a micro-processor, or programmed electronic circuitry, and/or a computer system (collectively and/or separately referred to as a computer system, for simplicity) equipped with a tangible non-transitory storage medium. An optical illumination system is configured to produce a light output at an output end of the illumination system, and includes a source of light but does not include (that is, is devoid of) an apparatus that is configured to maintain a power of the light output to be substantially constant over time. (Such apparatus may include electronic circuitry controlled by a processor, a neutral density filter, and acousto-optical modulator - to name just a few.) An optical imaging (or light collecting) system contains an optical detector system. A computer system is operably connected with the optical detector system and configured to receive electrical signals therefrom representing a speckle image formed by said optical imaging system in light from said light output. A computer-readable tangible non-transitory medium is configured to contain a computer-readable program code disposed on which are stored computer-readable instructions such that, when the instructions are executed by a processor of the computer system, the instructions cause the processor at least to acquire, at only one fixed first empirical exposure time, one or more raw speckle images formed in said light by the imaging system; and to spatially average a chosen raw speckle image of said one or more raw speckle images with a use of multiple binning apertures that have spatial different dimensions to form respectively-corresponding modified speckle images, wherein each of said modified speckle images represents a speckle image corresponding to a respectively-corresponding second synthetic exposure time from a plurality of second synthetic exposure times. In at least one embodiment, the syMESI system may be configured to have each second synthetic exposure time from the plurality of second synthetic exposure times be necessarily different from one another and from the first empirical exposure time. Additionally or in the alternatively, and at least in one implementation, the instructions may be further configured to cause the processor to transform each of the modified speckle images into a respectively-corresponding speckle contrast image of a plurality of speckle contrast images corresponding to the same chosen image; and/or the instructions may be further configured to cause the processor (a) to display at least one of the one or more of raw speckle images, the chosen image, and at least one of the plurality of speckle contrast images as visually perceivable spatial distribution of optical irradiance, and/or (b) to determine and/or display a speckle visibility curve of the value of speckle contrast for at least one pixel of the given spectral contrast image as a function of second synthetic exposure times. In at least the latter case (and, optionally, in every implementation of the system), the instructions may be additionally configured to cause the processor to assess, based at least on the speckle visibility curve, a quantitative value of a motion at a portion of a scene irradiated with the light output in operation of the syMESI system and represented by one or more raw speckle images. Alternatively or in addition, and substantially regardless of the specific implementation of the syMESI system the instructions may be additionally configured to further cause the processor to generate a visually perceivable image of a portion of a scene irradiated with said light output in operation of the syMESI system and represented by said one or more raw speckle images, wherein said visually perceivable image displays a spatial distribution of a quantitative value of a motion at said portion of the scene via a spatial distribution of an optical parameter across said visually perceivable image. (Such optical parameter may irradiance in case of a substantially mono-chromatic image - for example, a bright field image, and/or a chosen color distribution within boundaries of the image in case of a polychromatic image, to name just a few.) Altenratively or in addition, and substantially in every implementation of the syMESI system, the instructions may be configured to cause the processor to acquire, at only one fixed first empirical exposure time, a sequence of raw speckle images formed in light produced by the imaging system (here, in at least one case, constituent raw speckle images in such sequence are made necessarily non-consecutive) and, whein constituent raw speckle images in such sequence are made necessarily non-consecutive, the optical imaging system may be configured to acquire such necessarily non-consecutive raw speckle images with time gaps of different durations in between immediately neighboring raw speckle images.

Embodiments additionally provide a method for characterizing a scene with the use of a syMESI system configured according to at least one of the embodiments identified above. Embodiments of the method include at least irradiating the scene with a first light containing the light output from the optical illumination system; acquiring, at such only one fixed first empirical exposure time, one or more raw speckle images of the scene in a second light representing said first light backscattered by the scene; and - for each of a plurality of binning apertures that have different spatial dimensions - modifying a chosen image of the one or more raw speckle images into a corresponding one of multiple modified speckle images by spatially averaging an irradiance distribution of the chosen image with a respectively-corresponding binning aperture of the plurality of binning apertures, thereby producing a plurality of modified speckle images each of which represents a speckle image of the scene corresponding to a second synthetic exposure time of a plurality of second synthetic exposure times. Here, all second synthetic exposure times from the plurality of second exposure times are different from one another and from the first empirical exposure time. An embodiment of the method may additionally include a step of transforming each of the plurality of modified speckle images into a respectively-corresponding speckle contrast image of a plurality of speckle contrast images corresponding to the same chosen image. Alternatively or in addition - and in at least one implementation of the method - the first light may be chosen to be the light output from the imaging system; and/or the source of light may be chosen to be a laser source of light; and/or - for each of modified speckle image from the plurality of modified speckle images - a numerical relationship between the first empirical exposure time and the corresponding second synthetic exposure time may depend on a dimension of the predetermined binning aperture; and/or at least one of the one or more of raw speckle images, the chosen image, and at least one of the plurality of speckle contrast images may be made visually perceivable. Alternatively or in addition - and substantially in every implementation of the method - the at least one or more raw speckle images may be defined to include only one raw speckle image; or two raw speckle images of such at least one of more raw speckle images that are acquired consecutively may be defined to be acquired not immediately one after another but with an arbitrary time delay between such two images. Regardless of the specifics of a particular implementation - and substantially in every embodiment of the method - at least one of the following conditions may be satisfied: (a) the acquiring, at only one fixed first exposure time, one or more raw speckle images, includes acquiring only one raw speckle image; and/or (b) the step of modifying a chosen image of the one or more initial speckle images includes modifying of only one image of the one or raw speckle images. Similarly, alternatively or in addition, and in any chosen implementation of the method, each pixel of the given speckle contrast image may be defined to have a value of speckle contrast determined as a ratio of a standard deviation of respectively-corresponding pixel intensities of such modified speckle images to a mean of the intensities at hand. A binning aperture of the plurality of binning apertures may have a polygonal shape and, in a specific case, a shape of a concave polygon (as defined, for example, at mathopenref.com).

Furthermore - and substantially in every implementation of the method - the method may be configured to satisfy at least one of the following conditions: (i) the method further includes a step of quantitatively determining an index of motion at a portion of the scene represented by a given pixel of the chosen image; and (ii) the index of motion is an index of blood flow when the scene of interest scene is a biological tissue. In at least one implementation, the step of said acquiring may be configured to include acquiring multiple raw speckle images as with time-gaps between such images at said only one fixed first empirical exposure time, a sequence of raw speckle images formed with time gaps between such images (here, constituent raw speckle images in the sequence are chosen to be necessarily non-consecutive). In the latter case, the step of acquiring may include acquiring such necessarily non-consecutive raw speckle images with time gaps of different durations in between different immediately neighboring raw speckle images. Alternatively or in addition - and substantially in every implementation of the method, the step of modifying the chosen image for each of the plurality of binning apertures may be configured to include one of: - spatially averaging the irradiance distribution of the chosen image with the same binning aperture that is spatially repositioned across the chosen image; and - spatially averaging the irradiance distribution of the chosen image with multiple binning apertures of difference sizes and/or shapes (here, respectively corresponding reference corners of the apertures are fixed at the same location of the chosen image). Regardless of the specifics of a particular implementation of the method, each pixel of a given speckle contrast image produced by the method may be assigned a value of speckle contrast determined in a temporal domain, a spatial domain, or a spatio-temporal domain.

A method in accordance with the invention is defined in claim 1.

BRIEF DESCRIPTION OF THE DRAWINGS

For a more complete understanding of the disclosure, reference is made to the following detailed description and accompanying Drawings, of which:

FIGs. 1A, 1B, and 1C provide illustrations to the Laser Speckle Contrast Imaging (LSCI) methodology. Here, FIG. 1A presents a 'raw' speckle image acquired by camera. The raw image displays random interference pattern. FIG. 1B is a processed (on the basis of the image of FIG. 1A) 'speckle contrast' image that highlights spatial regions of high and low blood flow. FIG. 1C schematically illustrates a typical LSCI apparatus configured for *two-dimensional* imaging of cerebral blood flow.

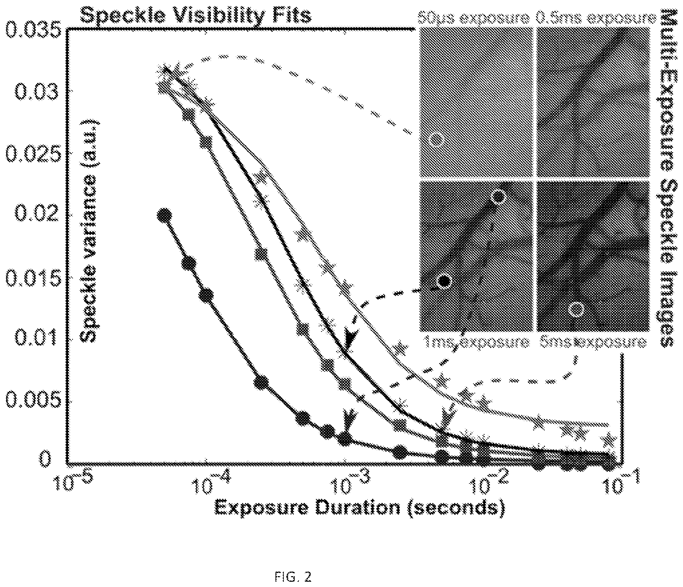

FIG. 2 illustrates the results of Multi-Exposure Speckle Imaging (MESI) of rat brain. Speckle variance as a function of camera exposure duration, T, is fit to the speckle visibility equation to derive quantitative estimates of CBF at four regions of interest. Images in inset are speckle contrast images of CBF in a mouse cortex. Camera exposure duration modulates the visibility of blood vessels.

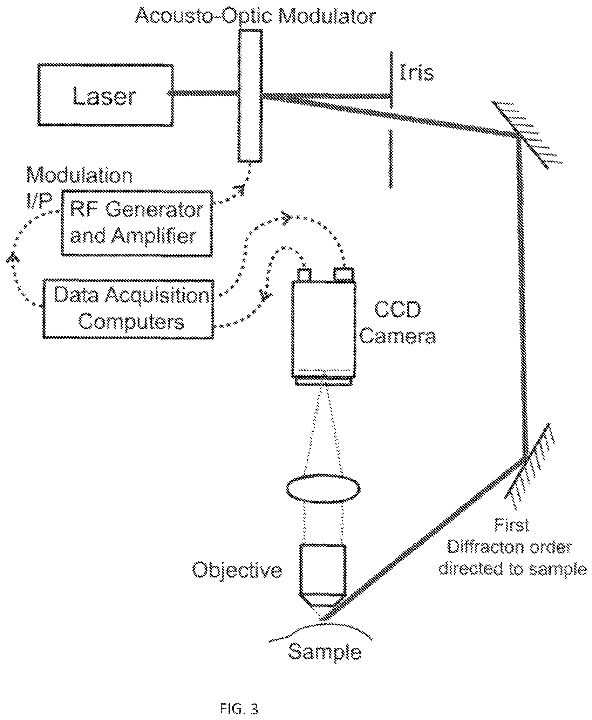

FIG. 3 schematically illustrates a typical setup for Multi-Exposure Speckle Imaging (MESI). An Acousto-Optic Modulator (AOM) is required to modulate the intensity of the laser over different exposure times. The camera and AOM are typically triggered with the data acquisition electronic circuitry (processor).

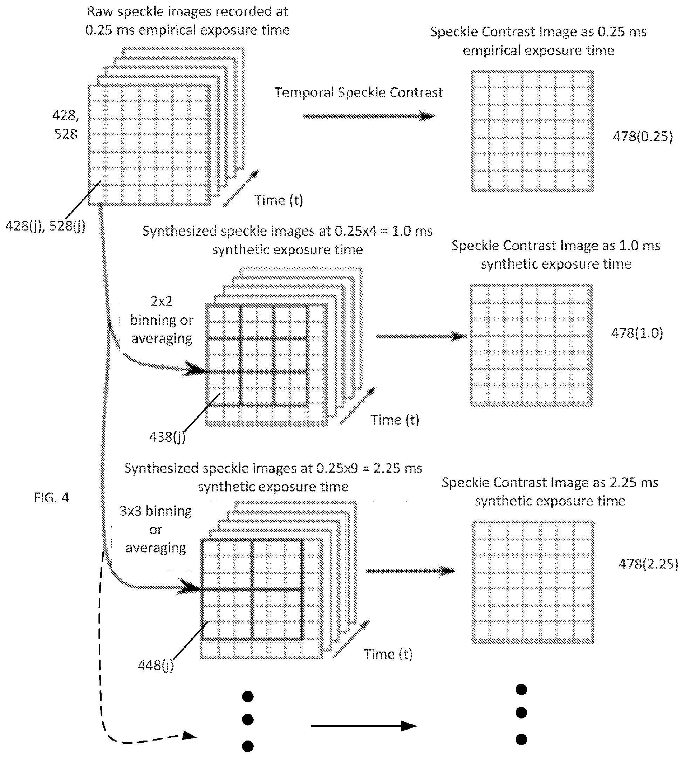

FIG. 4: *Synthetic* Multi-Exposure Speckle Imaging. Raw speckle images at longer exposure can be synthesized by spatially averaging images at shorter exposure. For example, speckle images at 1 ms exposure duration are *synthesized* by spatially averaging 4 pixels of a 0.25 ms speckle image. The averaging can be accomplished with a 2x2 moving window or by 2x2 binning. At each exposure duration, speckle contrast is computed at each image pixel in the *temporal* domain, by calculating the ratio of the standard deviation to mean of intensities.

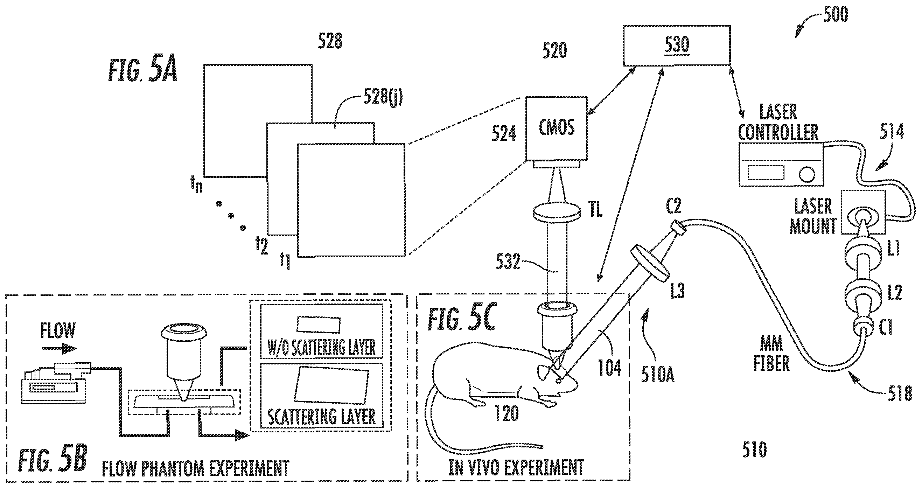

FIGs. 5A, 5B, 5C illustrate *Synthetic* Multi-Exposure Speckle Imaging methodology. FIG. 5A: Schematic of the imaging system; light from a single mode diode laser illuminates the sample. The back-reflected light is imaged using a standard imaging system and a CMOS camera. FIG. 5B: Microfluidic flow phantom for controlled flow experiments. Images were collected from two phantoms, one with a 200 $\mu m$ layer static scattering layer and the other without. FIG. 5C: *In vivo* experiments were performed to image cerebral blood flow on an anesthetized rat.

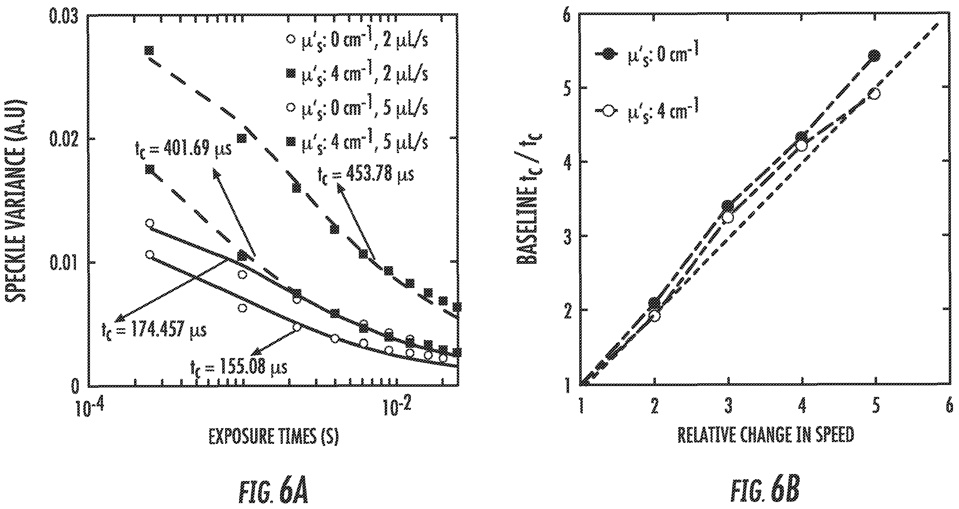

FIG. 6A presents Synthetic Multi-Exposure Speckle Contrast data acquired from two samples and fitted to the Multi-Exposure speckle model discussed in Ref. C. Shown is speckle variance as a function of exposure duration for two different speeds and two levels of static scattering. Solid lines represent measurements made on sample without static scattering layer. Dashed lines represent measurements made on sample with static scattering layer. $\mu'_s$ values refer to the reduced scattering coefficient in the 200 $\mu m$ static scattering layer. $\mu'_s = 0 \; cm^{-1}$: No static scattering layer. $\mu'_s = 4 \; cm^{-1}$: 0.9 mg/g of TiO$_2$ in static scattering layer. (see FIG. 5B).

FIG. 6B is a plot of relative correlation time to relative speed. Baseline flow: 1 $\mu L/sec$. The two curves shown represent different amounts of static scattering. New speckle model retains the linearity of relative $\tau_c$ estimates.

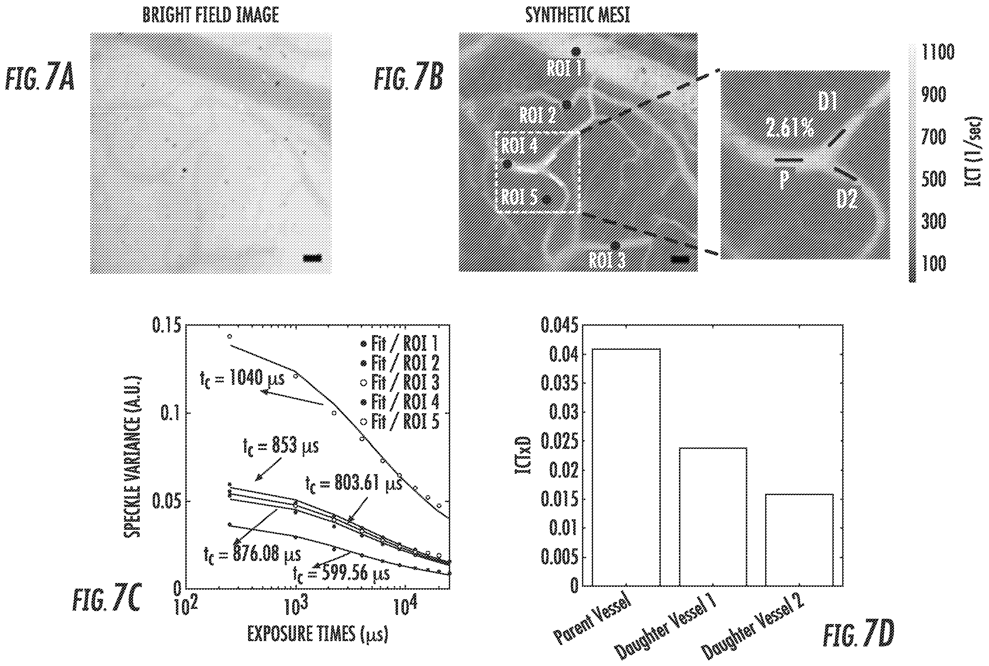

FIGs. 7A, 7B, 7C, 7D: Bright filed image of exposed rat cortex, FIG. 7A; Corresponding *synthetic* MESI image, FIG. 7B. Higher ICT ($1/\tau_c$) values indicate faster flow. FIG. 7C contains *synthetic* MESI-computed speckle visibility curves for the corresponding five ROIs from FIG. 7B. FIG. 7D illustrates conservation of flow analysis for three-way vessel branches, for the yellow rectangular ROIs. *Synthetic* MESI computed percentage of error due to the combination of flow from two daughter vessels (D1, D2) and the parent vessel (P) is shown in the inset of FIG. 7B. Scale bar: 100 $\mu m$.

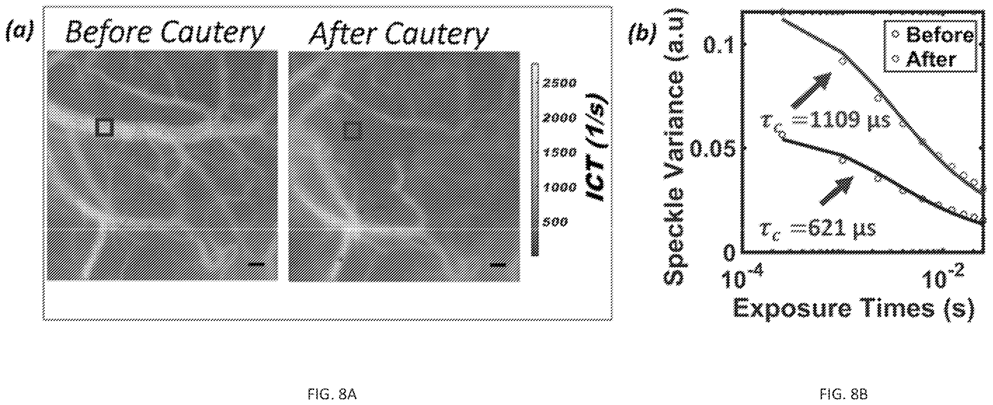

FIGs. 8A, 8B: *synthetic* MESI measured speckle inverse correlation time maps of flow before and after cautery of a cerebral blood vessel in an anesthetized rat (scale bar: 100 $\mu m$), FIGs. 8A; Corre-

sponding speckle visibility curves for the selected ROI highlighting a ~50% reduction in cerebral blood flow, FIG. 8B.

Generally, the sizes and relative scales of elements in Drawings may be set to be different from actual ones to appropriately facilitate simplicity, clarity, and understanding of the Drawings. For the same reason, not all elements present in one Drawing may necessarily be shown in another. While specific embodiments are illustrated in the figures with the understanding that the disclosure is intended to be illustrative, these specific embodiments are not intended to limit the scope of invention implementations of which are described and illustrated herein.

DETAILED DESCRIPTION

As was alluded to above, the currently-used in related art speckle imaging technique referred to as Multi-Exposure Speckle Imaging (or MESI), combines a more complex (as compared to that of the conventional LSCI system) instrument design with a new mathematical model for conversion of speckle contrast into quantitative indices of blood flow.

In performing the laser speckle imaging according to the MESI approach (see, e.g., Refs. A, B, and C), for example, the variance/mean values of instantaneous speckle intensity fluctuations are time-averaged over the durations of exposures of the camera, resulting in speckle contrast values that depend on the camera exposure duration. Stated differently, *the duration of* camera exposure effectively modulates the 'visibility' of blood vessels characterized by different flow rates: speckle contrast is more sensitive to fast intensity fluctuations at shorter exposures, while longer exposure durations are needed to detect slow intensity fluctuations. The earlier proposed MESI technique leverages this phenomenon and requires experimental recordation of multiple LSCI images (at camera exposures of different durations, for example at multiple durations from 50 $\mu$s to 80 ms, as shown in illustrations of FIG. 2). In practice, such leveraging is accomplished by externally triggering the camera acquisition and the laser light source while ensuring that power level of irradiation of the sample is kept substantially constant across the dynamic range of the measurement (see Ref. C). The relationship between speckle contrast and camera exposure duration can be characterized by a speckle visibility equation which in its simplest form is given by: $K(T, \tau_c) = \beta(e^{-2x} - 1 + 2x)/2x^2$. Here, $x = T/\tau_c$, $\beta$ is a speckle instrumentation factor that depends on wavelength of light, detector pixel size and speckle size, T is the camera exposure duration and $\tau_c$ is the speckle decorrelation time - a quantitative index that is inversely proportional to blood flow. $\beta$ and $\tau_c$ are estimated from a nonlinear curve fit (see FIG. 2) of the multi-exposure-time speckle data (that is, speckle data acquired by the optical detector at multiple exposure times independently) to the speckle visibility expression. This multi-exposure-time MESI methodology was shown to enable the determination of $\beta$, which acts as an instrument and sample dependent calibration factor, thereby permitting imaging of baseline/absolute blood flow. *In situ* measurements of $\beta$ can also be used to quantitatively estimate blood flow index ($\tau_c$) from single exposure LSCI measurements. MESI was demonstrated to correct underestimation of large CBF changes (see Refs. A, C) and with a robust speckle visibility expression, reduced the sensitivity of speckle imagers to static tissue elements such as the skull. The advanced instrument design and model discussed in Refs. A, C (the necessary part of which is a device configured to maintain the light output, directed onto the target subject to examination from the illumination portion of the instrument, at a constant level) have dramatically improved speckle-based imagers, enabling for the first-time, quantitative chronic comparisons of CBF images on the same animal, and intra-subject comparisons.

Recently, the MESI approach has been extended by related art to deep-tissue blood flow measurements with Diffuse Speckle Contrast Analysis. These studies utilize 'speckle-contrast' processing schemes (DSCA) to measure tissue dynamics from diffusely reflected light, recorded by integral detectors such as large CCD/CMOS cameras, SPAD arrays, or EMCCD cameras. Essentially, they measure speckle intensity fluctuations (*i.e., It(T)*) at multiple detector integration times, fit the resultant speckle variance to a diffuse speckle contrast model to estimate blood flow.

Notably, the conventional multi-exposure speckle imaging (MESI) approach used in related art thus far necessarily requires the acquisition of images of speckle intensity at multiple different camera exposures (that is, at multiple exposures of different durations), since such imaging methodology is turning on and therefore necessarily involves time-averaging of speckle contrast values. A person of skill immediately appreciates the practical shortcoming of the multi-exposure-time MESI methodology: acquisition of images over a large dynamic range of exposure times (for example, from 50 $\mu$s to 80 ms) is a non-trivial instrumental challenge. Indeed, since speckle contrast is computed as the variance of intensities normalized to their mean, the user of MESI must somehow maintain the average intensity of the laser beam reaching the target tissue substantially constant across a very large range of camera exposures. This condition, however, cannot be satisfied with the use of electronic control of the laser current because, at larger exposure times, the current required to maintain low optical power of the laser falls below the lasing threshold. FIG. 3 illustrates one common implementation of the conventional MESI approach where an acousto-optic modulator (AOM) is used to control the intensity of the laser light source used in LSCI. (Alternatively, an array of neutral density filters can be used to control the optical power of the illumination, as was discussed in Ref. D).

As the skilled person now understands, the experimentally desired temporal resolution afforded by the use of the multi-exposure-time time-averaging conventional MESI methodology is rooted in and provided by the ability to control the optical power of the laser light impinging on the target (interrogated with light) scene (for example - biological tissue). This need to control the optical power of the laser leads to at least two major shortcomings of the currently employed MESI approach. First, instrumentation to accomplish control of illumination intensity adds complexity to the system (even and especially when compared with the simpler, conventional LSCI approach). This substantially prevents the implementation of multi-frame time-averaging MESI method in challenging environments such as for intraoperative blood flow imaging or retinal blood flow imaging, makes instrument alignment difficult, and prevents usage of the system in low-light conditions. Additionally or alternatively, the need to acquire images over a large range of exposure times (up to 80 ms) necessarily and inevitably reduces the effective image acquisition speed to only a few Hz, thereby reducing the fidelity of imaging of fast blood flow dynamics (such as blood pulsation, rapid functional activations, and cortical spreading depressions, to name a few).

Embodiments of the current invention - referred to as a "synthetic MESI" (or, syMESI) methodology - solve the problems associated with the multi-exposure-time time-averaging MESI-based implementation of the LSCI by simultaneously avoiding the need to control power of laser light irradiating the target scene (and thus allowing the user to revert to the practical use of the basic, simpler LSCI system schematically depicted in, for example, FIG. 1) and, at the same time, providing the quantitative assessment of the particle movements in the target that the conventional, single-exposure-time LSCI approach is not capable of providing. It is understood that implementations of the discussed idea of the invention are directed to non-intrusive imaging of and determining a motion present at various objects - be it an inanimate object or space containing certain moving portions or elements or living objects such as a biological tissue hosting a flow of blood - all of which are within the scope of the invention. That said, the discussion of embodiments of the invention below is presented with the specific non-limiting examples of a target that is a biological tissue, for simplicity and certainty of presentation.

As was already alluded to above, implementations of the proposed "synthetic Multi-Exposure Speckle Imaging" (syMESI) technique are configured to result in formation of the same speckle images that the conventional, multi-exposure-time MESI methodology delivers by empirically capturing fluctuations of speckle intensity of light delivered from the target scene over a broad range of durations of the camera exposures (often, from 50 μs to 80 ms) - except without the use of imaging at multiple exposure times and without time-averaging of the empirically-acquired images. Instead, in advantageous contradistinction with the conventional MESI technique, the syMESI reimagines acquisitions of multi-exposure-time images in the spatial domain rather than in the time domain and, accordingly, does not require and is free from using a component or element or device configured to maintain the level of light output (produced by an illumination portion of the syMESI system for the purposes of the irradiation / illumination of the target scene) constant, temporally unchanged.

The idea of the invention stems from the realization that that since speckle is a random process that satisfies ergodicity conditions, and since speckle intensities or values of irradiance or power received and acquired at spatially adjacent pixels of a camera (an optical detector system) are independent of each other. speckle images of a scene that would otherwise require a practical "capture" by the optical detection system at various desired exposure times can be generated or synthesized (without having the optical detector of the optical detection system or camera being actually exposed to the incoming light at such various desired exposure times) by spatially averaging of image(s) empirically acquired at a certain exposure time that is shorter than any of these desired exposure times. For example, a speckle image at 4 ms exposure can be formed by transforming (via, for example, spatially averaging 4 pixels of) an image empirically acquired by exposing the detector to incoming light at 1 ms exposure time. So-formed speckle images (that can be interchangeably referred to as multi-synthetic-exposure-time speckle images or as synthetic multi-exposure-time speckle images) can be readily formed for different synthetic (that is, not implemented in the process of light-acquisition by the optical detector of the overall system) exposure times from the same set of raw speckle images that includes at least one raw speckle image.

Accordingly, in one embodiment, the syMESI apparatus of the disclosure includes an optical illumination system, and optical imaging system, and a computer system coordinating the operation of the syMESI apparatus.

FIGs. 5A, 5B, 5C schematically illustrate the syMESI apparatus or system 500 that, for practical purposes, is substantially similar to a conventional LSCI apparatus of FIG. 1C with a substantial and advantageous difference: the system 500 is configured to provide quantitative measurements of the motion present at the target (scene, sample) 120 - which the conventional LSCI apparatus is not configured to carry out. Here, the optical illumination system 510 (of the apparatus 500) having the output end 510A is shown to include the source of light 514 (in one case - containing the laser source of light with an appropriate controller / driver and supported, in needed, with a dedicated harness structure) and an optical delivery system 518 transferring light from the source of light 514 to then output end 510A to generate the light output 104. In the example 500, the optical delivery system includes, as shown, a system of lens elements and an optical fiber element (but of course such delivery system an be appropriately modified to serve specific requirements of a specific measurement). An optical imaging system 520 (containing, in addition to some optical relay elements such as lens elements, the optical detector or optical detection system 524 labelled as "CMOS" in FIG. 5A), is disposed in optical communication with the optical illumination system 510. The optical imaging system 510 is configured to acquire a set of raw speckle images of the scene 120, irradiated with light output 104. According to the idea of the invention, the set of raw speckle images may include one or more of raw speckle images and is taken at only one, single value of the time exposure of the optical detector of the optical detector system 524. When the set of raw speckle images includes multiple raw speckle images 528(j) (the plurality 528 of which is indicated in FIG. 5A), the constituent individual raw speckle images are taken or acquired without any pre-requisite requirement with respect to the corresponding time of acquisition. In one specific case, for example, the time gaps or delays between the immediately-neighboring constituent images of the plurality 528 can be different from one another. Unlike the conventional MESI methodology, the implementation of the proposed syMESI does not require a consecutive acquisition of raw speckle images. (For the purposes of this disclosure, and unless expressly identified otherwise, the term "consecutive" and related terms are defined to identify items or processes that are following substantially continually, and that have substantially no temporal interruptions in between.) In one specific implementation, the system 500 is configured to acquire (at only one pre-determined fixed exposure time) a sequence of raw speckle images (formed by the imaging system 524 in light generated by the illumination system 510 - as shown, in a portion 532 of light 104 backscattered by the scene 104) in which constituent raw speckle images are necessarily non-consecutive.

The dedicated (preferably programmable) electronic circuitry such as a microprocessor or a processor of a computer system, indicated in FIG. 5A as 530, is appropriately equipped with program code configured to govern the operation of various subsystems of the apparatus 500 (including, for example, a repositioning stage on which the target / scene 120 is disposed; not shown in FIG. 5A) and to acquire and appropriately process the optical imaging information. In particular, the apparatus 500 is configured to operate to acquire, at only one fixed empirical exposure time, one or more raw speckle images formed in light generated by the optical illumination system 510; and to spatially average a chosen raw speckle image of such one or more raw speckle images with a use of multiple binning apertures that have spatial different dimensions - thereby forming respectively-corresponding modified speckle images each of which represents a speckle image corresponding to a respectively-corresponding second - this time, synthetic - exposure time. Each of the values of synthetic exposure times is different from another synthetic exposure time and from the first (empirical) exposure time at which the image set 528 is acquired with the optical imaging system 520.

An example of the *synthetic* Multi-Exposure Imaging procedure, configured according to the idea of the present invention, is schematically outlined in FIG. 4. As shown, at least one or a series of multiple raw speckle images 428, 528 are acquired by the camera at an only, single, fixed exposure time (empirical acquisition of raw speckle data; here - at 0.25 *ms),* using the conventionally-structured traditional LSCI instrumentation (e.g., FIG. 1C or FIG. 5A). At one, chosen or - alternatively - each of these images is then modified / transformed / converted to corresponding *synthetic* multi-exposure-time images (that is, images corresponding to multiple synthetic exposure times) by spatially averaging the camera recorded chosen image 528(j) with an appropriate spatial window or aperture (interchangeably - binning aperture). The shape of the aperture is preferably polygonal (considering the pixelated nature of raw speckle images 528) and, in a specific case, the averaging spatial aperture may have a shape of a concave polygon - that is, a polygon at least one angle of which exceeds 180 degrees. Here, as shown in the example of FIG. 4, spatially averaging the chosen raw speckle image 528(j) taken at an empirical exposure time of 0.25 ms with a $2 \times 2$ square window / binning aperture yields a modified image that is a *synthetic* exposure image 438(j). This image 438(j) is a speckle image of the scene 120 corresponding to a synthetic (not empirical) exposure time of 1 *ms.* Each pixel of the *synthetic 1* ms image 438(j) is an average of 2 $\times$ 2 = 4 adjacent pixels of the camera-acquired 0.25 ms empirical exposure time image 428(j), 528(j). In this manner, FIG. 4 also illustrates how a modified speckle image 448(j) corresponding to a synthetic exposure time of 2.25 ms can be formed . By repeating this process multiple times - as indicated in FIG. 4 with ellipses - with variously-shaped and/or sized binning windows for at least one (and, if needed, more than one) empirically recorded raw speckle image from the set 428, the user or the system produce a plurality of modified speckle images each of which represents a speckle image of the scene corresponding to a corresponding synthetic exposure time (of a plurality of decided upon synthetic exposure times).

Additionally - and referring again to FIG. 4-the information about the motion present at the imaged scene 120 i.e. blood flow information in the biological tissue, is quantified by determining the temporal speckle contrast at each image pixel for all synthetic exposure times, *i.e.,* by computing $K = \sigma_s / \langle I \rangle$ at each pixel of the, where $\sigma_s$ is the standard deviation and $\langle I \rangle$ is the mean of pixel intensities of n modified speckle images (typically, n = 30~50 synthetic image frames). Thereby, the set(s) of modified speckle images 438(j), 448(j), and so on is transformed to a respectively-corresponding images containing information about speckle contrast. See the speckle contrast image 478(0.25) representing the spatial distribution of speckle contrast within bounds of the empirically-acquired at the exposure time of 0.25 ms image(s) 428, 528. See also the speckle contrast image 478(1.0) representing the spatial distribution of speckle contrast within bounds of the generated, by spatial binning average, synthetic image(s) 438(j) corresponding to synthetic exposure time of 1.0 ms. See also the speckle contrast image 478(2.25) representing the spatial distribution of speckle contrast within bounds of the generated, by spatial binning average, synthetic image(s) 448(j) corresponding to synthetic exposure time of 2.25 ms. Furthermore, at each pixel of such speckle contrast image(s) one can thus assess the speckle visibility (expressed in at least one example as a plot or curve of speckle variance vs synthetic exposure time, e.g., FIG. 2) that can further be fit to either a single-scattering quantitative speckle contrast spectroscopy model (see Refs. A, C) or a diffuse-scattering quantitative speckle contrast spectroscopy model (see Refs. E, F) to estimate an index of motion at a portion of the interrogated scene 120 represented by a given pixel of the chosen raw speckle image (and, in the case when the scene is a biological tissue - an index of blood flow at the corresponding portion of the tissue).

Notably, the spatial averaging can be performed using a variety of approaches as befitting the application - including pixel-wise binning (expressly depicted in FIG. 4) with spatial windows or apertures of various sizes and/or shapes placed substantially in the same location of the chosen raw speckle image, or with a spatial window or aperture of a fixed size and/or shaped that is repositioned across the chosen raw speckle image. The latter can retain the high spatial resolution of the images. These operations can be performed using standard image processing algorithms. Furthermore, as the skilled artisan has already appreciated from the discussion above, the spatial averaging is not restricted to a rectangularly-shaped spatial window or binning aperture. For example, with reference to FIG. 4, a 3x2 spatial window can be used to synthesize $0.25 \times 6 = 1.5$ ms exposure image. As applications require, windows of arbitrary (optionally-polygonal) shapes and sizes can be used.

The following provides an example of image acquisition / image formation / image transformation steps in an embodiment of the synthetic MESI invention:

a) Setup imaging camera and initialize system.
b) Acquire one or more raw speckle images with the camera; $I(x, y, n, T_{acq})$ - $n^{th}$ camera image $I(x, y)$ acquired at time $t$ and pre-defined single empirical exposure time $T_{acq}$.
c) Define spatial window/aperture shape and size depending on the required $i^{th}$ synthetic exposure time ($T_i$) and the empirical exposure time $T_{acq}$:

$$\text{Window area } A = \frac{T_i}{T_{acq}}$$

Window size p $\times$ q such that p $\times$ q = A; for rectangular/square windows p = q $\approx round(A/2)$.
d) Generate spatial averaging window (kernel) $h(p, q)$; the magnitude of each pixel = 1/A.
e) Form modified speckle images - synthetic speckle image(s) $I(x, y, n, T_i)$ by spatially averaging (e.g., via spatial binning) the chosen of empirically-acquired with the imaging camera (optical detector) raw speckle. For example, determine the convolution of the empirically-acquired raw speckle image and the kernel; $I(x, y, n, T_i) = I(x, y, n, T_{acq}) \otimes h(p, q)$. Spatial averaging can also be performed by methods such as spatial binning, or any algorithm that performs a local mean of intensities in an image.
f) Repeat steps c) and d), if required, for all predetermined synthetic exposure times $T_i$.
g) Repeat steps b) to f), if required till end of image transformation session.

The following steps h), i), j) can be optionally completed either offline (post-image-processing) or simultaneously with steps a) through g) for real-time computing of quantitative maps (spatial distributions) of motion at the imaged scene":

h) Compute the temporal speckle contrast image at each exposure time

$$K(x, y, n, T_i) = \frac{\sigma(x,y,n,T_i)}{\langle I(x,y,n,T_i)\rangle}.$$

Here, $\sigma(x, y, n, T_i)$ may be computed as the standard deviation of pixel values in images $I(x, y, n - n_{avg}, T_i)$ to $I(x, y, n + n_{avg}, T_i)$; $\langle I(x, y, n, T_i)\rangle$ may be computed as the mean of pixel values in images $I(x, y, n - n_{avg}, T_i)$ to $I(x, y, n + n_{avg}, T_i)$; and both $\sigma(x, y, n, T_i)$ and $\langle I(x, y, n, T_i)\rangle$ can be computed with a moving temporal window or, alternatively, with a moving spatial window, or in a spatio-temporal fashion,
i) Determine and/or display, if desired, speckle visibility curves $(v(x, y, n, T_i) = K^2(x, y, n, T_i))$ for each pixel $x, y$ and each image frame $n$, as a function of synthetic exposure time $T_i$.
j) If desired, fit speckle visibility curves from step i) to a quantitative speckle contrast spectroscopy motion models (blood flow models, in one non-limiting example) to estimate indices of motion (such as indices of blood flow) $(F(x, y, n))$ for each pixel $x, y$ and each image frame n.

Having the advantage of the above disclosure, the skilled person will now readily appreciate various operational advantages of the proposed methodology over the existing systems and methods used in related art. The approach to use spatial averaging to *synthesize* (as opposed to practically acquire) multi-exposure-time speckle images naturally lends itself to several advantages over comparable instruments (and, in particular, in the field of imaging / measuring blood flow):

- The instrumentation for the *synthetic* MESI approach is extremely simple; the instrument requires only a CMOS/CCD camera and illumination from aa single mode laser. Any camera with such as conventionally available image sizes (as little as 1 megapixels), bit depths and light sensitivities can be used. There are no requirements for specialized sensor technologies (such as APD arrays), cooling or image intensifications - the technique will be shot-noise limited.

- No special equipment such as acousto-optic modulators, filter wheels or pulsed lasers are required to generate the multi-exposure images.

- Only a single exposure time imaging is involved: Multiple speckle images corresponding to different exposure images are not practically acquired but generated as a result of transformation of one or more of empirically acquired raw speckle image(s), for each recorded camera frame. A high frame rate camera is not required for the technique to work. As a result, the proposed approach can be successfully used to perform quantitative video rate multi-exposure speckle imaging (about 10 times faster than current state of the related art).

The range of synthetic exposure times for generation of the speckle visibility curves can be readily tuned per needs of the application. The only requirements is the predetermination of the duration of the only, single empirical exposure time of the camera for acquisition of the at least one raw speckle image.

The algorithm can be applied retrospectively previously recorded speckle images at a given empirical exposure time.

**Discussion of Experimental Results:**

Referring again to FIGs. 5A, 5B, 5C, one feature of multi-exposure speckle imaging is its ability to image baseline blood flow, especially in the presence of superficial static scattering layers. The first experiment (see setup in FIG. 5A) validated that images transformed according to the proposed *synthetic* MESI methodology successfully reproduced the capabilities of a traditional, multi-exposure-time MESI instrument. Briefly, a linearly polarized light output 104 from a single mode laser diode ($\lambda$ = 785 *nm,* 90 mW, Thorlabs) was collimated to substantially uniformly illuminate a tissue simulating microfluidic flow phantom (FIG. 5B). Backscattered light 532 from the sample 120 was imaged using a custom system that included a CMOS camera (Basler acA2000-165um-

NIR) and an objective lens (4X, NA 0.10, Olympus). A sequence of raw speckle images was recorded at a frame rate of 100 fps at an (empirical) exposure time of 250 $\mu s.$ Synthetic MESI images and speckle visibility curves were derived with approaches discussed above for synthetic exposure times of 250 $\mu s$ to 25 *ms. Two different samples of* tissue simulating flow phantoms - one with a 200 $\mu m$ thick static scattering layer ( $u'_s = 4\,cm^{-1}$ ) and the other without (see FIG. 5B) were used. Raw speckle images were collected while flowing 20% intralipid through a 200 $\mu$m $\times$ 150 $\mu$m flow channel, at flow rates 1 to *5 $\mu$L/s.*

The following two important results from the microfluidic phantom experiment (in reference to FIGs. 6A, 6B) are worth noting:

(1) Quantitative imaging of flow in the presence of static scatterers is shown in FIG. 6A, which illustrates the speckle visibility curves obtained from a region within the flow channel; these are fit to a quantitative speckle flow model for two flow rates (blue/red lines), for the two phantoms (solid/dashed lines). The results of the fit show that embodiments of the proposed *synthetic* MESI approach successfully reproduce the quantitative flow imaging first reported with traditional MESI (see 18). Notably, the estimated speckle decorrelation times estimated for each flow rate are similar, irrespective of the presence of the static layer. *Synthetic* MESI reproduces the classic change in shape of speckle visibility curves due to the presence of static scatterers, and the data fits well to the traditional MESI model. Other features of traditional MESI are reproduced; for a given exposure duration and speed, the measured speckle contrast values are different in the presence of static scattered light when compared to the speckle contrast values obtained in the absence of static scattered light. As with the traditional MESI approach, *synthetic* MESI addresses this concern by generating synthetic multiple exposure images, measuring and fitting the speckle visibility curves to reproduce $\tau_c$ (a flow index) consistently.

(2) Linearity of large flow changes in presence of static scatterers (FIG 6B) Here, it was also verified that *synthetic* MESI methodology affords the quantitative measurement of large relative flow changes in the presence of static scatterers. To this end, the speckle decorrelation times for different rates flow in the microfluidic phantom were computed, with and without static scatterers. Speckle visibility curves were computed and $\tau_c$ was estimated for each flow rate. The relative change in flow (baseline $\tau_c$ / measured $\tau_c$) was estimated and plotted as a function of relative flow rate as set by the syringe pump. FIG. 6B provides clear evidence, appreciated by a skilled person, that *synthetic* MESI proposed in this disclosure accurately mapped relative flow on a linear scale, and that it retains the linearity of relative correlation time measures even in the presence of static scatterers (similar to traditional, multi-empirical-exposure-time MESI). This again reinforced the fact that the *synthetic* MESI algorithm can predict consistent correlation times in the presence of static scatterers.

Another experiment was performed *in vivo,* to demonstrate the utility of *synthetic* MESI. A rat (Brown-Norway, male, 400-450 g) was anesthetized with an intraperitoneal injection of ketamine hydrochloride (75 mg/kg) and xylazine (7.5 mg/kg), supplemented as needed, and a craniotomy was performed to expose the cerebral vasculature. *Synthetic* MESI images of the vasculature were acquired before and after electrical cautery of a cerebral blood vessel. The animal was sacrificed after the experiment. *In vivo* experiments were performed with protocols approved by the Institutional Animal Care and Use Committee at the University of South Florida.

Notable are two important results from *in vivo* experiment (see FIGs. 7A, 7B and inset, 7C, and 7D):

(A) The ability to measure quantitative blood flow. FIGS. 8A through 7D present baseline *in vivo* cerebral blood flow images recorded from a rat brain. A 'bright field' image and corresponding *synthetic* MESI speckle inverse correlation time (ICT) image ($1/\tau_c$) is shown in FIGs. 7A and 7B, respectively. Speckle ICTs are a flow index that is linearly proportional to blood flow. To appreciate the blood flow rates in vessels of different sizes and tissues of different types, five regions of interest (ROIs) are selected as in the synthetic MESI ICT frame. FIG. 7C shows the speckle variance curve for each of the ROI. These curves demonstrate that $\tau_c$ obtained by *synthetic* MESI follows expected physiological trends. The speckle visibility curves for ROI 1 (largest vessel) decay faster than curves for ROIs 2-4 (smaller vessels) and ROI 5 (parenchyma); the corresponding $\tau_c$ values increase from ROI 1 to ROI 5. Flow conservation analysis were also performed to further validate that *synthetic* MESI measure blood flow quantitatively. Here, speckle ICT values were computed in a parent vessel (P) and two daughter vessels (D1 and D2) as shown in the inset of FIG. 7B. Flow conservation were computed as the product of ICT and vessel diameter; the flow in the parent vessel was found to be the sum of the flows in the daughter vessels to within 2.6%.

B) Ability to measure quantitative blood flow changes (see FIGs. 8A, 8B). Here, *synthetic* MESI imaging was performed before and after cautery of a cerebral blood vessel to illustrate the feasibility of clinical imaging blood flow changes during surgery. FIG. 8A shows, in two parts, synthetic MESI ICT images before and after cautery of a cerebral vessel. After cautery *synthetic* MESI clearly shows reduction of flow in the location of cautery. Tissue locations away from the cautery retains their flow with minimal changes. FIG. 8B shows the speckle variance curves before and after cautery for the selected ROI; a 50 % reduction in cerebral blood flow can be observed due to cautery.

Now the skilled person can appreciate that the proposed *synthetic* MESI methodology can be readily used in a wide variety of applications. First, the approach can be applied for a variety of applications that utilize laser speckle contrast imaging for blood flow imaging, including, but not limited to imaging of skin microvascular function and dysfunction, wound healing angiogenesis, diagnosis of tissue burns, skin cancer, endoscopic surgical procedures and GI tract surgery, ulcer, cardiovascular studies, diabetes, and cerebrovascular studies. More generally, the *synthetic* MESI approach can be applied to quantify speckle fluctuation dynamics in any multi-speckle detection system, including those applied for Diffuse Correlation Spectroscopy, laser speckle rheology and speckle-based thrombosis measurements.

Contents of each of related art references and/or articles identified in this disclosure is incorporated herein by reference.

References throughout this specification to "one embodiment," "an embodiment," "a related embodiment," or similar language mean that a particular feature, structure, or characteristic described in connection with the referred to "embodiment" is included in at least one embodiment of the present invention. Thus, appearances of the phrases "in one embodiment," "in an embodiment," and similar language throughout this specification may, but do not necessarily, all refer to the same embodiment. It is to be understood that no portion of disclosure, taken on its own and in possible connection with a figure, is intended to provide a complete description of all features of the invention.

Within this specification, embodiments have been described in a way that enables a clear and concise specification to bet written, but it is intended and will be appreciated that embodiments may be variously combined or separated without parting from the scope of the invention. In particular, it will be appreciated that all features described herein at applicable to all aspects of the invention.

In addition, when the present disclosure describes features of the invention with reference to corresponding drawings (in which like numbers represent the same or similar elements, wherever possible), the depicted structural elements are generally not to scale, and certain components are enlarged relative to the other components for purposes of emphasis and understanding. It is to be understood that no single drawing is intended to support a complete description of all features of the invention. In other words, a given drawing is generally descriptive of only some, and generally not all, features of the invention. A given drawing and an associated portion of the disclosure containing a description referencing such drawing do not, generally, contain all elements of a particular view or all features that can be presented is this view, at least for purposes of simplifying the given drawing and discussion, and directing the discussion to particular elements that are featured in this drawing. A skilled artisan will recognize that the invention may possibly be practiced without one or more of the specific features, elements, components, structures, details, or characteristics, or with the use of other methods, components, materials, and so forth. Therefore, although a particular detail of an embodiment of the invention may not be necessarily shown in each and every drawing describing such embodiment, the presence of this particular detail in the drawing may be implied unless the context of the description requires otherwise. In other instances, well known structures, details, materials, or operations may be not shown in a given drawing or described in detail to avoid obscuring aspects of an embodiment of the invention that are being discussed. Furthermore, the described single features, structures, or characteristics of the invention may be combined in any suitable manner in one or more further embodiments.

For the purposes of this disclosure and the appended claims, the use of the terms "substantially", "approximately", "about" and similar terms in reference to a descriptor of a value, element, property or characteristic at hand is intended to emphasize that the value, element, property, or characteristic referred to, while not necessarily being exactly as stated, would nevertheless be considered, for practical purposes, as stated by a person of skill in the art. These terms, as applied to a specified characteristic or quality descriptor means "mostly", "mainly", "considerably", "by and large", "essentially", "to great or significant extent", "largely but not necessarily wholly the same" such as to reasonably denote language of approximation and describe the specified characteristic or descriptor so that its scope would be understood by a person of ordinary skill in the art. In one specific case, the terms "approximately", "substantially", and "about", when used in reference to a numerical value, represent a range of plus or minus 20% with respect to the specified value, more preferably plus or minus 10%, even more preferably plus or minus 5%, most preferably plus or minus 2% with respect to the specified value. As a non-limiting example, two values being "substantially equal" to one another implies that the difference between the two values may be within the range of +/- 20% of the value itself, preferably within the +/- 10% range of the value itself, more preferably within the range of +/- 5% of the value itself, and even more preferably within the range of +/- 2% or less of the value itself.

The use of these terms in describing a chosen characteristic or concept neither implies nor provides any basis for indefiniteness and for adding a numerical limitation to the specified characteristic or descriptor. As understood by a skilled artisan, the practical deviation of the exact value or characteristic of such value, element, or property from that stated falls and may vary within a numerical range defined by an experimental measurement error that is typical when using a measurement method accepted in the art for such purposes.

The term "image" generally refers to an ordered representation of detector output corresponding to spatial positions. For example, a visual image may be formed, in response to a pattern of light detected by an optical detector, on a display device X such as a video screen or printer. The term "quantitative" is defined as that which is or may be represented by quantity.

The invention as recited in claims appended to this disclosure is intended to be assessed in light of the disclosure as a whole, including features disclosed in related art to which reference is made.

While the invention is described through the above-described exemplary embodiments, it will be understood by those of ordinary skill in the art that modifications to, and variations of, the illustrated embodiments may be made without departing from the inventive concepts disclosed herein. Disclosed aspects, or portions of these aspects, may be combined in ways not listed above. Accordingly, the invention should not be viewed as being limited to the disclosed embodiment(s).

## Claims

1. A synthetic Multi-Exposure Imaging method comprising:

   acquiring one or more raw speckle images (528) by illuminating a sample (120) with divergent light (104) from a laser source of light (514);
   collecting backscattered light (532) through an optical imaging system (520), and recording speckle intensity as raw speckle images on a camera sensor (524) at a fixed first empirical exposure time;
   performing data processing by a processor (530), wherein the data processing comprises: converting the raw speckle images to synthetic multi-exposure images (438(j), 448(j)) by spatially averaging a chosen one of the one or more camera sensor recorded images (528(j)) with a corresponding spatial window, thereby synthesizing multi-exposure speckle images, wherein the averaging comprises using multiple binning apertures that have different spatial dimensions to form respectively-corresponding modified synthetic speckle images, wherein each of said modified synthetic speckle images represents a speckle image corresponding to a respectively-corresponding second synthetic exposure time from a plurality of second synthetic exposure times, wherein the plurality of second synthetic exposure times corresponds to a multiplicity of the multiple binning apertures, and wherein each second synthetic exposure time from the plurality of second synthetic exposure times is different from one another and from the fixed first empirical exposure time; and

quantifying blood flow information by computing temporal speckle contrast at each image pixel for all exposure times by computing $K = \sigma s/(I)$ at each pixel, where $\sigma s$ is the standard deviation and $(I)$ is the mean of pixel intensities of n sequential modified synthetic speckle images.

2. A method according to claim 1, further comprising: transforming each of the plurality of modified speckle images into a respectively-corresponding speckle contrast image of a plurality of speckle contrast images corresponding to the same one chosen image of the raw speckle images.

3. A method according to one of claims 1 and 2, wherein:
   for each of said modified speckle images from the plurality of modified speckle images, a numerical relationship between the first empirical exposure time and the corresponding second synthetic exposure time depends on a spatial dimension of said predetermined binning aperture

4. A method according to one of claims 2 to 3, wherein each pixel of the given speckle contrast image has a value of speckle contrast determined as a ratio of a standard deviation of respectively-corresponding pixel intensities of said modified speckle images to a mean of said intensities.

5. A method according to one of claims 1 to 4, wherein said averaging includes one of:
   spatially averaging an irradiance distribution of the chosen raw speckle image with the same binning aperture of the multiple binning apertures that is spatially repositioned across the chosen raw speckle image; and
   spatially averaging the irradiance distribution of the chosen raw speckle image with the multiple binning apertures of difference sizes and/or shapes, wherein respectively corresponding reference corners of said multiple binning apertures are fixed at the same location of said chosen raw speckle image.

**Patentansprüche**

1. Verfahren für synthetische Mehrfachbelichtungs-Bildgebung, umfassend:

Erfassen eines oder mehrerer roher Speckle-Bilder (528) durch Beleuchten einer Probe (120) mit divergentem Licht (104) von einer Laserlichtquelle (514);
Sammeln von rückgestreutem Licht (532) durch ein optisches Bildgebungssystem (520) und Aufzeichnen der Speckle-Intensität als rohe Speckle-Bilder auf einem Kamerasensor (524) zu einer festen ersten empirischen Belichtungszeit;
Durchführen von Datenverarbeitung durch einen Prozessor (530), wobei die Datenverarbeitung umfasst:

Konvertieren der rohen Speckle-Bilder in synthetische Mehrfachbelichtungsbilder (438(j), 448(j)) durch räumliches Mitteln eines gewählten der einen oder mehreren von dem Kamerasensor aufgezeichneten Bilder (528(j)) mit einem entsprechenden räumlichen Fenster, wodurch Mehrfachbelichtungs-Speckle-Bilder synthetisiert werden, wobei das Mitteln das Verwenden mehrerer Binning-Aperturen umfasst, die unterschiedliche räumliche Abmessungen aufweisen, um jeweils entsprechende modifizierte synthetische Speckle-Bilder zu bilden, wobei jedes der modifizierten synthetischen Speckle-Bilder ein Speckle-Bild repräsentiert, das einer jeweils entsprechenden zweiten synthetischen Belichtungszeit aus einer Vielzahl von zweiten synthetischen Belichtungszeiten entspricht, wobei die Vielzahl von zweiten synthetischen Belichtungszeiten einer Multiplizität der mehreren Binning-Aperturen entspricht, und wobei jede zweite synthetische Belichtungszeit aus der Vielzahl von zweiten synthetischen Belichtungszeiten voneinander und von der festen ersten empirischen Belichtungszeit verschieden ist; und
Quantifizieren von Blutflussinformationen durch Berechnen des zeitlichen Speckle-Kontrasts an jedem Bildpixel für alle Belichtungszeiten durch Berechnen von $K = \sigma s/(I)$ an jedem Pixel, wobei $\sigma s$ die Standardabweichung ist und $(I)$ der Mittelwert von Pixelintensitäten von n sequenziellen modifizierten synthetischen Speckle-Bildern ist.

2. Verfahren nach Anspruch 1, ferner umfassend:
   Transformieren jedes der Vielzahl von modifizierten Speckle-Bildern in ein jeweils entsprechendes Speckle-Kontrastbild einer Vielzahl von Speckle-Kontrastbildern, die demselben einen gewählten Bild der rohen Speckle-Bilder entsprechen.

3. Verfahren nach einem der Ansprüche 1 und 2, wobei:

für jedes der modifizierten Speckle-Bilder aus der Vielzahl von modifizierten Speckle-Bildern eine numerische Beziehung zwischen der ersten empirischen Belichtungszeit und der entsprechenden zweiten synthetischen Belichtungszeit von einer räumlichen Abmessung der vorbestimmten Binning-Apertur abhängt.

4. Verfahren nach einem der Ansprüche 2 bis 3, wobei jedes Pixel des gegebenen Speckle-Kontrastbildes einen Wert des Speckle-Kontrasts aufweist, der als ein Verhältnis einer Standardabweichung von jeweils entsprechenden Pixelintensitäten der modifizierten Speckle-Bilder zu einem Mittelwert der Intensitäten bestimmt wird.

5. Verfahren nach einem der Ansprüche 1 bis 4, wobei das Mitteln eines der Folgenden beinhaltet:

räumliches Mitteln einer Bestrahlungsstärkeverteilung des gewählten rohen Speckle-Bildes mit derselben Binning-Apertur der mehreren Binning-Aperturen, die über das gewählte rohe Speckle-Bild hinweg räumlich neu positioniert wird; und
räumliches Mitteln der Bestrahlungsstärkeverteilung des gewählten rohen Speckle-Bildes mit den mehreren Binning-Aperturen von unterschiedlichen Größen und/oder Formen, wobei jeweils entsprechende Referenzecken der mehreren Binning-Aperturen an derselben Stelle des gewählten rohen Speckle-Bildes fixiert sind.

## Revendications

1. Procédé d'imagerie multi-exposition synthétique comprenant :

l'acquisition d'une ou de plusieurs images à granularité brute (528) en éclairant un échantillon (120) avec une lumière divergente (104) provenant d'une source de lumière laser (514) ; la collecte de la lumière rétrodiffusée (532) à travers un système d'imagerie optique (520) et l'enregistrement de l'intensité de granularité sous forme d'images à granularité brute sur un capteur de caméra (524) à un premier temps d'exposition empirique fixe ;
la réalisation d'un traitement de données par un processeur (530), dans lequel le traitement de données comprend :

la conversion des images à granularité brute en images synthétiques multi-exposition (438(j), 448(j)) par calcul de moyenne spatiale d'une image choisie parmi les une ou plusieurs images enregistrées par le capteur de caméra (528(j)) avec une fenêtre spatiale correspondante, synthétisant ainsi des images à granularité multi-exposition, dans lequel le calcul de moyenne comprend l'utilisation de plusieurs ouvertures de regroupement ayant des dimensions spatiales différentes pour former des images à granularité synthétique modifiée correspondantes, dans lequel chacune desdites images à granularité synthétique modifiée représente une image à granularité correspondant à un second temps d'exposition synthétique respectivement correspondant parmi une pluralité de seconds temps d'exposition synthétique, dans lequel la pluralité de seconds temps d'exposition synthétique correspond à une multiplicité des plusieurs ouvertures de regroupement et dans lequel chaque second temps d'exposition synthétique parmi la pluralité de seconds temps d'exposition synthétique est différent les uns des autres et du premier temps d'exposition empirique fixe ; et
la quantification d'informations sur le flux sanguin en calculant le contraste de granularité temporel à chaque pixel d'image pour tous les temps d'exposition en calculant $K = \sigma s/(I)$ à chaque pixel, où $\sigma s$ est l'écart type et $(I)$ est la moyenne d'intensités de pixels de n images à granularité synthétique modifiée séquentielle.

2. Procédé selon la revendication 1, comprenant en outre :
la transformation de chacune de la pluralité d'images à granularité modifiée en une image de contraste de granularité respectivement correspondante parmi une pluralité d'images de contraste de granularité correspondant à la même image choisie parmi les images à granularité brute.

3. Procédé selon l'une des revendications 1 et 2, dans lequel :
pour chacune desdites images à granularité modifiée parmi la pluralité d'images à granularité modifiée, une relation numérique entre le premier temps d'exposition empirique et le second temps d'exposition synthétique correspondant dépend d'une dimension spatiale de ladite ouverture de regroupement prédéterminée.

4. Procédé selon l'une des revendications 2 à 3, dans lequel chaque pixel de l'image de contraste de granularité donnée a une valeur de contraste de granularité déterminée comme un rapport entre un écart type d'intensités de pixels respectivement correspondantes desdites images à granularité modifiée et une moyenne desdites intensités.

5. Procédé selon l'une des revendications 1 à 4, dans lequel ledit calcul de moyenne comporte l'un des éléments suivants :

le calcul de moyenne spatiale d'une distribution d'éclairement de l'image à granularité brute choisie avec la même ouverture de regroupement que les plusieurs ouvertures de regroupement qui sont repositionnées spatialement sur l'image à granularité brute choisie ; et le calcul de moyenne spatiale de la distribution d'éclairement de l'image à granularité brute choisie avec les plusieurs ouvertures de regroupement de tailles et/ou de formes différentes, dans lequel des coins de référence respectivement correspondants desdites plusieurs ouvertures de regroupement sont fixés au même emplacement de ladite image à granularité brute choisie.

'Raw' Speckle Image

(A)

High Contrast (low flow)

Low Contrast (high flow)

FIG. 1A

Window

(C)

$$K = \frac{\sigma}{\langle I \rangle}$$

(B)

FIG. 1B

Speckle Contrast Image

CCD Camera

To Computer    FIG. 1C

130

110
Diode laser

To diode controller

Zoom lens

128

Collimation optics

124

100

104

Sample 120

FIG. 2

FIG. 3

Raw speckle images recorded at
0.25 ms empirical exposure time

Speckle Contrast Image as 0.25 ms
empirical exposure time

428,
528

Temporal Speckle Contrast

478(0.25)

428(j), 528(j)

Time (t)

Synthesized speckle images at 0.25x4 = 1.0 ms
synthetic exposure time

Speckle Contrast Image as 1.0 ms
synthetic exposure time

2x2
binning or
averaging

478(1.0)

438(j)

Time (t)

Synthesized speckle images at 0.25x9 = 2.25 ms
synthetic exposure time

Speckle Contrast Image as 2.25 ms
synthetic exposure time

FIG. 4

3x3 binning
or
averaging

478(2.25)

448(j)

Time (t)

20

FIG. 5A

FIG. 5B FLOW PHANTOM EXPERIMENT

FIG. 5C IN VIVO EXPERIMENT

FIG. 6A

FIG. 6B

EP 4 312 743 B1

BRIGHT FIELD IMAGE

*FIG. 7A*

SYNTHETIC MESI

*FIG. 7B*

ROI 1

ROI 2

ROI 4

ROI 5

ROI 3

D1
2.61%
P
D2

ICT (1/sec)

1100
900
700
500
300
100

*FIG. 7C*

$t_c = 1040 \ \mu s$

$t_c = 853 \ \mu s$

$t_c = 803.61 \ \mu s$

$t_c = 876.08 \ \mu s$

$t_c = 599.56 \ \mu s$

• Fit / ROI 1
• Fit / ROI 2
○ Fit / ROI 3
• Fit / ROI 4
○ Fit / ROI 5

SPECKLE VARIANCE (A.U.)

0.15

0.1

0.05

0

$10^2$  $10^3$  $10^4$

EXPOSURE TIMES ($\mu s$)

ICT×D

0.045
0.04
0.035
0.03
0.025
0.02
0.015
0.01
0.005
0

Parent Vessel    Daughter Vessel 1    Daughter Vessel 2

*FIG. 7D*

FIG. 8A

FIG. 8B

EP 4 312 743 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63200914

### Non-patent literature cited in the description

- **PARTHASARATHY et al.** Robust flow measurement with multi-exposure speckle imaging. *Optics Express*, 2008, vol. 16, 1975-1989
- **PARTHASARATHY, A. B.** ; **KAZMI, S. M. S.** ; **DUNN, A. K**. Quantitative imaging of ischemic stroke through thinned skull in mice with Multi Exposure Speckle Imaging. *Biomedical optics express*, 2010, vol. 1, 246-259
- **KAZMI, S. M. S.** ; **PARTHASARATHY, A. B.** ; **SONG, N. E.** ; **JONES, T. A.** ; **DUNN, A. K**. Chronic imaging of cortical blood flow using Multi-Exposure Speckle Imaging. *Journal of Cerebral Blood Flow & Metabolism*, 2013, vol. 33, 798-808
- **PARTHASARATHY, A. B.** ; **TOM, W. J.** ; **GOPAL, A.** ; **ZHANG, X.** ; **DUNN, A. K**. Robust flow measurement with multi-exposure speckle imaging. *Optics Express*, 2008, vol. 16, 1975-1989
- **RICHARDS, L. M. et al.** Intraoperative multi-exposure speckle imaging of cerebral blood flow. *Journal of Cerebral Blood Flow & Metabolism*, 2017, vol. 37, 3097-3109
- **VALDES, C. P. et al.** Speckle contrast optical spectroscopy, a non-invasive, diffuse optical method for measuring microvascular blood flow in tissue. *Biomedical optics express*, 2014, vol. 5, 2769
- **VARMA, H. M.** ; **VALDES, C. P.** ; **ADAMS, K. E.** ; **CULVER, J. P.** ; **DURDURAN, T**. Speckle contrast optical tomography: A new method for deep tissue three-dimensional tomography of blood flow. *Biomedical optics express*, 2014, vol. 5, 1275
- **DRAGOJEVIC et al.** Compact, multi-exposure speckle contrast optical spectroscopy (SCOS) device for measuring deep tissue blood flow. *Biomedical optics express*, 2018, vol. 9, 322